# NEW EUROPEAN PATENT SPECIFICATION

(11) **EP 0 153 926 B2**
(45) Date of publication and mention of the opposition decision: **10.03.1999**
(45) Mention of the grant of the patent: 06.02.1991
(21) Application number: 84902489.8
(22) Date of filing: 14.06.1984
(51) Int. Cl.: A61K 9/22, A61K 9/52, A61K 9/42, A61K 31/685, A61K 47/00

(54) **MICRODROPLETS OF WATER-INSOLUBLE DRUGS**
MIKROTROPFEN WASSERUNLÖSLICHER ARZNEIMITTEL
MICRO-GOUTTELETTES DE MEDICAMENTS INSOLUBLES DANS L'EAU

(30) Priority: 17.06.1983 US 505326
(43) Date of publication of application: 11.09.1985
(73) Proprietor: PHARMA-LOGIC, INC., Miami, Florida 33133 (US)
(72) Inventor: Haynes, Duncan Harold, Miami, Florida 33133 (US)
(74) Representative: Goldin, Douglas Michael
(86) International application number: PCT/US84/00906
(87) International publication number: WO 85/00011

(56) References cited:
- EP-B- 0 052 322
- DE-A- 2 513 797
- DE-P- 2 938 807
- US-A- 3 137 631
- US-A- 3 594 476
- US-A- 3 715 432
- US-A- 3 755 557
- US-A- 3 937 668
- US-A- 4 073 943
- US-A- 4 145 410
- US-A- 4 147 767
- US-A- 4 241 046
- US-A- 4 271 196
- US-A- 4 302 459
- US-A- 4 308 166
- US-A- 4 320 121
- US-A- 4 328 222
- US-A- 4 331 654
- US-A- 4 351 831
- US-A- 4 356 167
- US-A- 4 369 182
- US-A- 4 394 372
- US-A- 4 397 846
- US-A- 4 448 765
- Römpp's Chemie Lexikon, 2. Aufl. B.1, 1950, Stichwort "Emulsion"
- Der Ultraschall (Ludwig Bergmann), 5. Aufl. 1949, Stuttgart, S. 551-564, 672f
- Kirk-Othmer, Encyclopeida of Chemical Technology, 3rd Ed. (1978), vol. 2, pp. 684-699
- Dictionary of medecine, Dr.Med. J. Nöhring, Verlag Harri Deutsch, 1984, p. 244
- Rote Liste 1982, Nr 64002 bis 64006

## Description

Microdroplets, originally called monolayer vesicles, were previously used to study the properties of the phospholipid surface as a model for the true phospholipid vesicle which, in turn, was a model for the biological membrane. This approach is to be distinguished from liposomes (multilamellar-) and unilamellar phospholipid vesicles used to deliver water-soluble drugs to the interior of cells, both *in vivo* and *in vitro*. These liposomes are true vesicles and consist of a spherical lipid bilayer with an aqueous phase inside.

Microdroplets are known and consist of spheres of organic fluid phase drug approximately 500 Angstroms in diameter and are covered with a monolayer of a suitable phospholipid.

The present invention provides a microdroplet consisting essentially of a substantially water-insoluble sphere of liquid anaesthetic surrounded by a phospholipid layer as defined in the claims. The microdroplet of the invention can be defined more precisely as one of from about 20 nm (200 Ångströms) up to one µm in diameter consisting essentially of a sphere of a substantially water-insoluble anaesthetic dissolved in a compatible, pharmaceutically acceptable organic liquid selected from an alkane, a dialkyl ether, a long-chain ester, a hydrophobic ester, a biocompatible silicone, a biocompatible high molecular weight fluorocarbon, an oil-soluble vitamin and a volatile liquid anesthetic, the liquid plus anaesthetic being surrounded by a layer of phospholipid.

The microdroplets of the invention can be presented essentially as a timed release drug delivery vehicle composed of microdroplets consisting essentially of a substantially water-insoluble anaesthetic, itself a liquid or dissolved in a water-insoluble liquid, stabilized against coalescence and surrounded by a phospholipid layer.

A process for producing microdroplets consisting essentially of a substantially water-insoluble anaesthetic, compatible pharmaceutically-acceptable organic liquid and a surrounding lipid layer that stabilizes the microdroplets against coalescence, can comprise:
(1) preparing a homogenized suspension of the microdroplet components including the water-insoluble anaesthetic and phospholipid where the ratio of anaesthetic to phospholipid is sufficient to form a layer of said microdroplet and subjecting the homogenized suspension to sonification for a time sufficient to produce a cloudy, stable suspension of microdroplets; or
(2) preparing a homogenized suspension of the microdroplet components including the water-insoluble anaesthetic and phospholipid, wherein the ratio of anaesthetic to phospholipid is sufficient to form a layer on said microdroplets and subjecting the homogenized suspension to high intensity mechanical agitation or shear to produce a cloudy, stable suspension of microdroplets; or
(3) dissolving the microdroplet components including the water-insoluble anaesthetic, organic liquid and phospholipid in an oil and water miscible organic solvent to form a solution, diluting the solution with a physiological saline solution with vigorous mechanical agitation to dissolve the solvent in the organic phase and to allow the finally dispersed constituents spontaneously to form microdroplets and removing the solvent; or
(4) preparing a homogenized suspension of the microdroplet components including the water-insoluble anaesthetic and phospholipid, wherein the ratio of drug substance to phospholipid is sufficient to form a layer on the microdroplet and spraying the homogenized suspension into physiological saline solution.

In a still further embodiment of the invention, there is provided the use of a normally volatile known general anaesthetic in liquid form for the manufacture of a medicament for use in producing local anaesthesia in a warm-blooded animal by injection. Further specific embodiments are characterised by the dependent claims.

The microdroplets of the invention can be used to deliver any water-insoluble/oil-soluble anaesthetic compound via injection. Non-polar anaesthetics now taken orally are contemplated and are within the scope of the invention. The organic liquid phase may be the anaesthetic itself. The advantages of the microdroplets provided by the invention include a relatively slow release of the anaesthetic to the tissues and allow for a targeted delivery by intelligent choice of the site of injection with lowered metabolic degradation, first pass effects, and toxic side-effects in the liver an the other organs.

Local anesthesia is conventionally accomplished by injection of water-soluble compounds into the site to be anesthetized.

In contrast to local anesthesia is general anesthesia, which is accomplished by inhalation of anesthetic gases to produce unconsciousness. These include nitrous oxide, halothane®, isofluorane, enfluorane and methoxyfluorane, The first-named example is a true gas; the others are volatile fluoro-chloro-hydrocarbons which exist in liquid form: Liquid general anesthetics are water-insoluble and immiscible. They are volatized in to the air which the patient breathes, they gain access to the circulation through the lungs and cause unconsciousness by binding to the nerve membranes in the brain.

The novelty of one embodiment of the invention lies in the fact that it uses general anesthetics as local anesthetics. According to a current popular conception of physicians and biomedical scientists the use of inhalation anesthetics as local anesthetics is impossible. The textbooks and scientific papers deal with the local anesthetics and the general (often termed "volatile" and "inhalation" anesthetics) as separate classes of drug substances. According to contemporary thought this division is correct since the volatile anesthetics exist as oil-like liquids which are impossible to inject due to their low solubility in water-injection as such would be unthinkable. Injection of a liquid phase of any of the volatile anesthetics would result in membrane delipidation, cellular damage and eventual tissue necrosis. Dilution of such agents in saline is not feasible because of their water-insolubility. Yet it is this low water-solubility and high solubility in the membrane phase which makes these agents effective blockers of nerve conduction in the brain (and elsewhere, but with less physiological consequence).

### Brief description of the drawings

Figure 1 is a perspective representation, partially broken away, of a microdroplet of the invention containing an organic liquid and an anaesthetic surrounded by a unimolecular lecithin outer surface;
Figure 2 is a graph based on the results of Example 1 comparing the percent response of 1% lidocaine over a period of up to 200 minutes following injection;
Figure 3 is a graph reporting the response for Example 1 as the percent response of rats to a pain stimulus induced by the tail-clamp technique, as a function of time after injection of microdroplets of methoxyfluorane;
Figure 4 is a graph based on Example 1 reporting the initial response in percent against the dose of methoxyfluorane, in volume percent; and
Figure 5 is a graph also based on Example 1 reporting the time necessary for recovery of 50% response after the injection of microdroplets of methoxyfluorane against concentration of microdroplets, in volume percent.

The uniqueness of the invention is that a means of reducing this liquid oil-like phase to microscopic droplets, for instance approximately 500 Ångstroms (estimated by calculation) in diameter is now available. Moreover, these microscopic droplets are stabilized against coalescence by a monolayer of phospholipid. Upon intradermal injection these microdroplets become entrapped in the interstitial space between cells and release their anesthetic in a slow and sustained manner. While not wishing to be bound to any particular theory or mode of operation, three possible mechanisms are prostulated for this: anesthetic diffusion, vesicle-cell membrane collision and fusion; see the discussion below. This is in contrast to normal elimination kinetics of an injected drug in which the drug is eliminated in a "first order" manner giving rise to an exponential decrease in concentration. With the controlled and sustained release, the concentration of the drug in the nerve and neighbouring tissue does not reach toxic concentrations. The rate of release can be controlled by the choice of anesthetic agent, based on vapor pressure and membrane solubility, and to some extent by the choice of lipid.

One skilled in the art following the instructions provided herein will have no difficulty in empirically determining an optimum relationship between anesthetic agent and compatible lipid coating. For the least exchangeable agents and most non-reactive lipids, the duration of effect will be governed by the time which it takes for the microdroplets to be cleared from the interstitial space and pass into the lymphatic system. The same principles are applicable to the use of lecithin-anesthetic microdroplets as a carrier for other water-insoluble drugs such as benzocaine or dantrolene.

Local anesthesia requires delivery of the drug directly to the nerve membrane. This requires that the drug be able to bind to membranes and to traverse lipid membranes, i.e., cell membranes, and that it be water-soluble and thus able to cross the aqueous regions between cells in order to diffuse to the nerve membranes. These requirements have been fulfilled by designing local anesthetics, for example procaine and lidocaine, which have both non-polar and polar structural features. Their water-solubility results in limitation of the life-time (duration) of anesthetic effect since the local anesthetics diffuse to capillaires and are removed by the blood in the above-mentioned first order process. Theoretically, this problem could be circumvented by employing local anesthetics which are poorly soluble in water, e.g., benzocaine, but the problem then becomes the delivery of the anesthetic. Water-insoluble local anesthetics are not absorbed well through the skin and it is not possible to inject them as one injects the water-soluble ones.

As mentioned above, general anesthetics are gases and volatile liquids which are inhaled to produce unconsciousness. They are poorly water-soluble compounds which enter the bloodstream by absorption in the lungs and which are carried through the bloodstream by binding to blood cells and proteins. They work on the central nervous system because it is most susceptible to their action, given this mode of delivery.

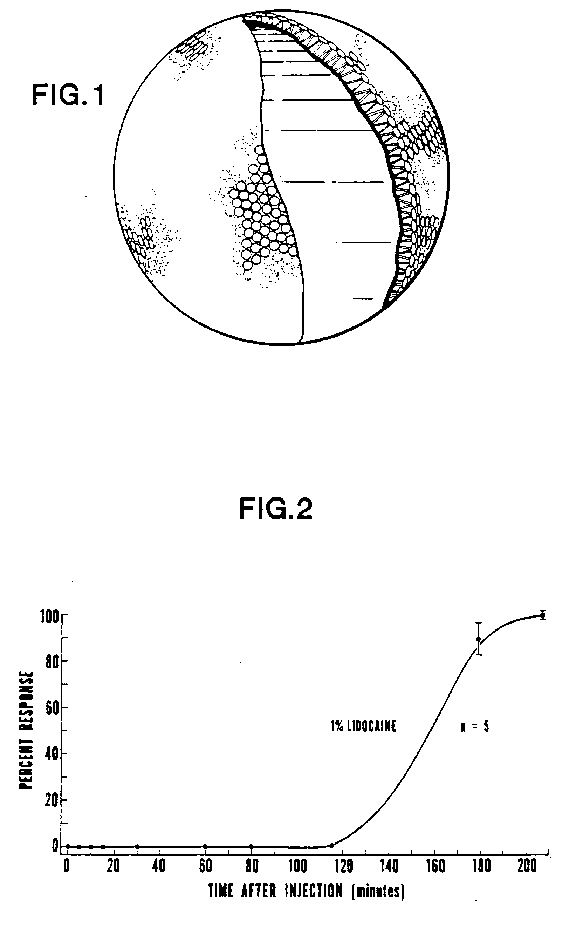

A microdroplet in accordance with the present invention is represented in perspective, partially broken away, in Figure 1, revealing a center containing the water-insoluble/organic phase containing the anaesthetic, surrounded by an outer unimolecular layer of lipid, such as lecithin. The properties of phospholipid membranes are described inter alia in my article concerning divalent cation-ligand interactions appearing in Metal-Ligand Interactions in Organic Chemistry and Biochemistry, Pullman and Goldblum, Part 2, pages 189―212, D. Reidel (1977).

One of the unique features of the invention lies in the use of volatile liquid general anesthetics to produce local anesthesia. Prior to this invention was not considered possible because it is not possible to inject an organic phase into the skin or other tissues without producing local damage due to dissolution of cell membranes and general derangement. Such a procedure would be literally unthinkable. The invention allows the injection of volatile general anesthetics without damage.

The key to accomplish the desired injection is to reduce the water-insoluble anesthetic (liquid) phase to microscopic dimensions, typically by sonication, and then coat the resulting structure with a layer of a lipid. Preferred are the phospholipids, which are natural constituents of biological membranes and as such are biologically compatible. A phospholipid is chosen which exhibits repulsive interaction with the cell membranes in the target tissue such that the microdroplet remains integral for the maximum time.

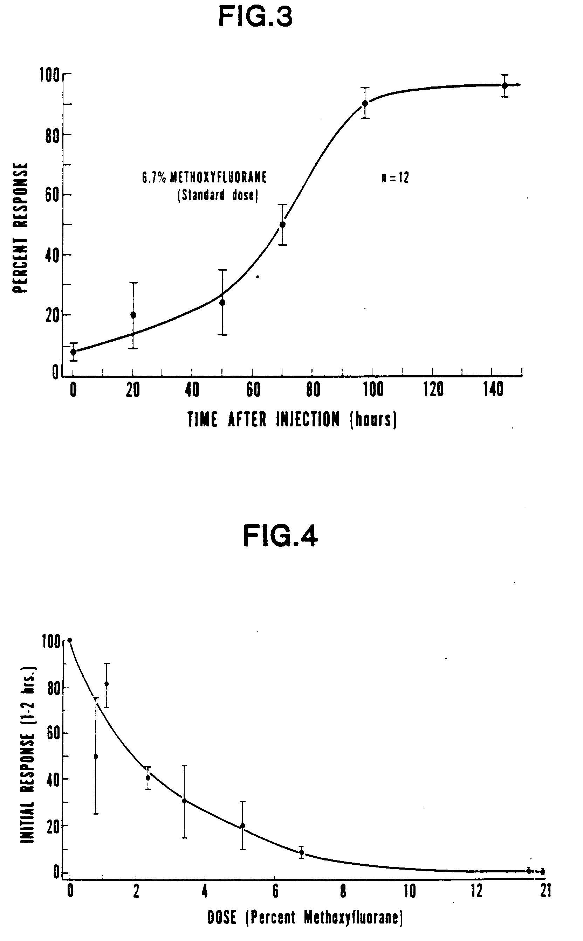

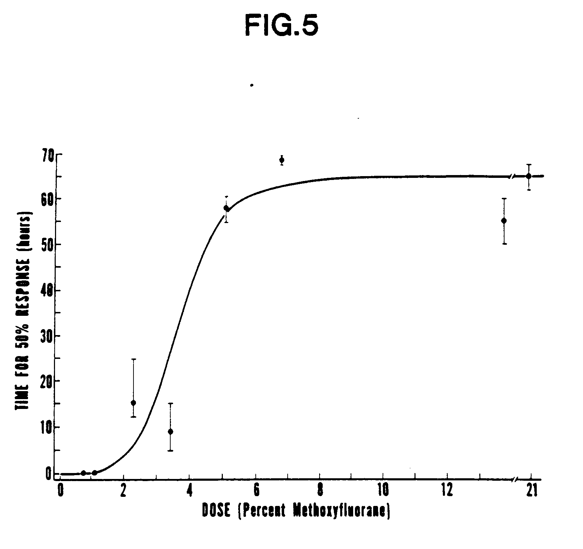

As mentioned above, it is believed that the microdroplets can transfer anesthetic to the tissue and nerves by three possible mechanisms: (a) solution, (b) collision/aggregation, or (c) fusion. Comparisons of anesthetic response plotted against time in hours after injection shown in Figure 3. From this and from Figures 4 and 5 it can be deduced that the release of the anesthetic from the microdroplets is slow and sustained. Figure 3 shows that the response rats to pain stimulus induced by tail clamping is decreased by injection of 0.5 ml of 6.7% methoxyfluorane microdroplets. The initial responsiveness (t=0―2½ hrs) is also dose-dependent as shown in Figure 4. The halftime for recovery of responsiveness increases with increasing anesthetic concentration, reaching a maximum of approximately 70 hours at high concentrations as shown in Figure 5. The above are illustrative and demonstrate effectiveness using three anesthetics, variable doses at a number of sites on the rat. Lidocaine was used as a control. Durations of lidocaine anesthesia were always less than 1/10th that of preparations in accordance with the invention.

While the research work leading to the recognition and completion of the present invention has been conceived primarily with anesthetics, and will in large part be illustrated and explained herein on that basis, the invention is not so limited and includes similar systems employing water-insoluble organic drug substances included in the unique drug delivery systems and procedures of my invention.

### Microdroplet preparation

The preferred method of preparing the microdroplets of the invention is by sonication with a probe sonicator. This is described in more detail below. Alternatively, microdroplets can be prepared in a bath sonicator. For small scale preparations a 1.0 cm diameter test tube is suspended, with use of a test-tube clamp, in a bath sonicator filled with water. The components of the microdroplet (organic phase, phospholipid, physiological saline, and anaesthetic to be included) are first grossly mixed by shaking, Vortex mixing, Polytron® or other methods. The homogenized suspension is then introduced into the bath sonicator and sonicated for 1―2 hours. If the preparation is to be done on a large scale, it will be possible to omit the test tube and introduce the components of the microdroplet directly into the bath sonicator.

Microdroplets may also be produced by high intensity mechanical agitation. Useful methods include the Waring® blender, the Polytron® and high frequency shakers such as a commercial paint shaker.

An aternative method to consider is the solvent dilution method. The desired constituents of the microdroplets are dissolved at high concentration in ethanol or another oil- and water-miscible organic liquid. The ethanol solution is rapidly diluted into the physiological saline solution with vigorous mechanical agitation to insure rapid mixing. The ethanol dissolves in the aqueous phase while the other constituents cannot. The finely-dispersed constituents spontaneously form microdroplets; the ethanol can be conveniently removed by dialysis.

Microdroplets can also be formed by a process similar to spray painting. The constituents of the microdroplets are suspended and sucked into intake of a commercial spray painter and the resulting spray bubbled through a saline solution to trap the microdroplets.

By judicious choice of methods and materials the diameter of the microdroplets is controlled between approximately 50 nm (500 Ångströms) to several micrometers by controlling the method, power and lipid to organic phase ratio. Increasing the power or the ratio tends to give smaller microdroplets. If natural or unsaturated lipids are used preparation is conducted in an atmosphere free from oxygen.

### Selection of organic phases

Microdroplets according to the present invention are prepared from a wide variety of organic phases which, for convenience, may be considered by the following non-limiting types or categories:
1. Volatile inhalation anesthetics include methoxyfluorane as well as halothane, isofluorane and enfluorane.
2. Alkanes include heptane, the heptane microdroplets can incorporate benzocaine which is suitable to produce long-duration local anesthesia. Higher molecular weight alkanes will also be potent. Mineral oil as the organic phase is also of interest as it is able to carry large quantities of water-insoluble drugs. Solubility may be increased by inclusion of more polar organic compounds with the alkane phase.
3. Ethers: Microdroplets have been made from dipropyl ether (3.4 mg/ml dimyristoyl lecithin, 6.5% n-dipropyl ether, ±4.1 mg/ml benzocaine) and dibutyl ether (5 mg/ml dimyristoyl lecithin, 7.0% n-dibutyl ether, ±4.1 mg/ml benzocaine). The dibutyl ether microdroplets and mixed dibutyl ether/chloroform microdroplets were found to have anesthetic potency. However, the anesthesia was of shorter duration (approximately ½ hour) possibly due to the greater water solubility of the dibutyl ether and chloroform which contributed to its more rapid removal. Longer-chain analogues could yield longer durations of activity.
4. Ethers: Any long-chain or hydrophobic ester is comtemplated particularly as a useful device for delivering "pro-drugs" which would be transformed into the active drug by hydrolysis by serum or cellular esterases.
5. Other organic substances which have been shown to be bio-compatible. These include by way of example silicone and high molecular weight fluorocarbons.

The organic phase selected will be fully compatible with the anaesthetic employed and pharmaceutically acceptable for product formulation/preparation purposes. As with all medical applications once the microdroplets are successfully prepared from a given organic phase and the selected anaesthetic incorporated therein, toxicological and efficacy screening is routine. Preferably the various components from which the microdroplets are made are subjected to toxicological screening as well.

### Lipids

Various lipids are also suitable for use in preparing the microdroplets of the present invention. Mixtures of two or more such lipids are useful to vary the surface properties and reactivity. All of the microdroplets in the working examples reported herein are made primarily from lecithin (phosphatidylcholine). This, together with sphingomyelin which is also contemplated, constitutes Class A. In Class B, are listed the phospholipids which can also be used to make microdroplets in the pure form, but which will react with calcium in the serum to give microdroplet aggregation or binding to cell membranes. The tendency to do this can be decreased by dilution with phosphatidylcholine, and thus there is a means of controlling the reactivity of the microdroplet. Class C contains only one representative, phosphatidylethanolamine. In the pure state it self-aggregates in a calcium-independent fashion. It is expected to have strong tendencies to aggregate with cell membranes. This tendency can be decreased by diluting it with lecithin. Class D, the steroids, do not form membranes or microdroplets by themselves, but which can be incorporated into the membrane, increasing its stability and decreasing its reactivity. Class E includes all molecules which can be accommodated in the monolayer. These are amphipathic molecules which can serve to change the nature of the monolayer surface and microdroplet reactivity.
Class A: Primary lipids (usable in pure form):
   Lecithin (phosphatidylcholine)
   Sphingomyelin.
Class B: These can be used in the pure form to make microdroplets (or phospholipid vesicles). They are expected to be highly reactive because of calcium-dependent aggregation. Preferably these lipids are mixed with lecithin to obtain controlled degrees of reactivity with cell membranes. Mixing in phospholipid vesicle preparations has already been demonstrated.
   Phosphatidic acid
   Phosphatidyl serine
   Phosphatidyl inositol
   Cardiolipin (diphosphatidyl glycerol)
   Phosphatidyl glycerol.
Class C: Phosphatidyl enthanolamine. This can be used to make microdroplets in the pure form at pH 9; they will self-aggregate when brought to pH 7. This has been shown to be feasible in phospholipid vesicle studies. Microdroplets made from phosphatidyl ethanolamine are expected to be very reactive with cell membranes. It is suggested that this lipid can be included with the normal lecithin to increase the reactivity to cell membranes.
Class D: Steroids: These should not be used in the pure form to make microdroplets but can be mixed with lecithin or other lipids to produce a surface which is less reactive, and presumably more stable.
   Cholesterol (natural constituent of membranes)
   Estrogens: Estirol, estrone, estradiol and diethylstilbestrol
   Androgens: Androstenedione, testosterone (the microdroplet would also constitute a delivery system for estrogens and androgens).
Class E: Semi-lipoidal molecules which can incorporate into the monolayer and change the surface activity of the microdroplet. These molecules could also be delivered to the nerve by the microdroplet.
   Stearylamine or other long-chained alkyl amines which can be primary, secondary, tertiary or quaternary substituted. These give the microdroplet surface a positive charge and make them more reactive for the cell membranes. These compounds could also be delivered to the nerve.
   Arachidonic acid or fatty acids. Could be incorporated into surface giving altered lipid packing and increased reactivity with cell membranes. The microdroplet is also a means of delivery of arachidonic acid for manipulations of prostaglandins.
Class F: Membrane-active agents, Nystatin, amphotericin B and gramacidin. These are surface-active antibiotics which have been shown to bind to the surfaces of phospholipid membranes and change their permeability. They are expected to change the reactivity of the microdroplet. The microdroplet is also a means of subcutaneous delivery of these antibiotics.

Several forms of lecithin are contemplated. For example lecithin is available as egg or bovine heart lecithin (natural) or in several synthetic varieties which differ in chain length. These include chain lengths ranging from 4 to 19 carbons (Supelco, Inc.). Dimyristoyl (14 carbons) and didodecanoyl (12 carbons) lecithin were used in the working examples (below). Didodecanoyl lecithin (12 carbons) may be considered more useful because the microdroplets will be more resistant to aggregation below room temperature. It is believed that lecithins with chain lengths in the biological range (10―18 carbons) are useful in various applications. Unsaturated lecithins (dioeoyl), dilinoeoyl; alpha-palmito, beta oleoyl; alpha palmitoyl beta linoleoyl and alpha oleoyl beta palmitoyl) are also available. Diarachidonyl lecithin (highly unsaturated and a prostaglandin precursor) is also available, as is alpha palmito beta myristoyl (mixed unsaturated chains) lecithin.

Phosphatidic acid is available from egg or as synthetic compounds (dimyristoyl, dipalmitoyl or distearoyl, Calbiochem). Bovine phosphatidyl serine is available (Supelco or Calbiochem).

Phosphatidyl inositol is available from plant (Supelco) or bovine (Calbiochem) sources. Cardiolipin is available (Supelco) from bovine or bacterial sources. Phospahtidyl glycerol is available from bacterial (Supelco) sources ir as synthetic compounds (dimyristoyl or dipalmitoyl; Calbiochem).

Phosphatidyl ethanolamine is available as egg, bacterial, bovine, or plasmalogen (Supelco) or as synthetic compounds dioctadecanoyl and dioleoyl analogues and dihexadecyl, dilauryl, dimyristoyl and dipalmitoyl (Supelco and Calbiochem).

### Anaesthetics

The following is a list of anaesthetics which may be incorporated into the microdroplets of the invention. This list is presented for purposes of illustration.
1. The volatile anesthetics are described above. They include methoxyfluorane, isofluorane, enfluorane and halothane®. Heptane was also shown to have anaesthetic potency.
   The following anaesthetics will be incorporated primarily in the organic phase of the microdroplet. They are all uncharged, lipophilic water-insoluble anaesthetics which have high oil solubility. In their applications, the organic phases of the microdroplets are made from the organic phase demonstrating the greatest drug solubility in macroscopic tests.
2. Dantrolene, a direct-acting muscle relaxant, is incorporated into methoxyfluorane microdroplets, heptane or mineral oil microdroplets. The resulting microdroplet suspension is injected around muscles and nerves for control of spasticity. This could circumvent the problem of hepatic toxicity seen with chronic oral administration of the drug.

All parts and percentages reported herein are by weight and all temperatures reported in degrees Celsius, unless otherwise indicated. The compositions of the invention can comprise, consist essentially of or consist of the materials set forth and the process or method can comprise, consist essentially of or consist of the steps set forth with such materials.

### Detailed description of the invention

### Example 1

Anesthetic-containing lecithin-coated micro-droplet are prepared by sonication in the following manner. Dimyristoyl phosphatidylcholine (41 mg) is added to a test tube and methoxyfluorane (0.2 ml) is pipetted in. The mixture is swirled in the tube at approximately 37°C and the lipid is observed to dissolve or be suspended to a limited extent. Next, sterile physiological saline (3.0 ml) is added and the tube is suspended under a Sonifier® Cell Disrupter, Model W185D (Heat System and Ultrasonics, Plainview, New York). The microtip is inserted and the sample is sonicated gently (power stage 2) for approximately one minute until the sample is dispersed. The oil, solid and aqueous phases are not distinguishable and gross homogeneity is obtained. The result appears as a milky single phase.

Next, the power is increased to stage 4 and the sample is sonicated for a total of approximately 5 minutes. The sonication temperature is between 30° and 45°C. The temperature can be controlled either by circulation of coolant around the sonication vessel or by interrupting the sonication periodically and allowing the sample to cool. The result of the sonication is a stable, homogenous suspension of lecithin-methoxyfluorane microdroplets. At the stated concentration, the suspension appears slightly cloudy to the eye; turbidity decreases with increasing dilution of the sample in accordance with Beer's Law. Efficacy and microdroplet properties do not depend on the concentration at which the microdroplets were prepared, as observed from experiments carried out over a wide range of concentrations. The preparation is stable for several days when stored at 30°C. The preparation retains the smell of methoxyfluorane indicating that the component is there and is releasable. Control experiments in which the lecithin is omitted from the medium failed to give microdroplets; phase separation was obtained immediately.

The efficacy of the preparation was tested with laboratory rats using a tail-clamp assay according to the method of Munson et al; (Munson, E.S., Hoffman, J.C. and DiFazio, C.A. "The Effects of Acute Hypothyroidism and Hyperthyroidism on Cyclopropane Requirement (MAC) in Rats" Anesthesiology 29; 1094―1098 (1968). The anesthetic preparation was injected into the tail and injections were distributed over four sites (0.5 ml total) such that a 3―4 cm long weal was obtained, encompassing all sides of the tail. Anesthesia was determined as being either present or absent from the response of the animal to clamping of the treated area with forceps as visually observed by squeeks or rapid movement. Untreated areas of the tail served as the control for the responsiveness of the animal to pain. As additional controls, some of the animals were injected with saline or sonicated lecithin without anesthetic agents. These controls showed uniformly no effect.

The efficacy of the microdroplet preparation was compared with that of 0.5 ml of 1% lidocaine (Figure 2) and bupivacaine in separate animals treated and tested in parallel. At least four animals were assigned to each treatment and dosage group. They were tested immediately after treatment and at timed intervals thereafter until complete repsonsiveness was obtained in all animals.

With lidocaine, the animals were rendered 0% responsive. On the time scale presented, the effect wore off rapidly. After 2.5 hours the animals were 50% responsive and no measurable effect is observed after six hours. A similar experiment was carried out using 0.5% bupivacaine which is the longest acting local anesthetic in clinical use. A similar response was observed (data not shown), the animals became 50% responsive after 6.5 hours and there was no measurable effect after 8 hours.

The results are shown in Figure 3 which illustrates the responsiveness of the 12 animals to the pain stimulus for the lecithin-methoxyfluorane microdroplet (1.28% lecithin, 6.25% methoxyfluorane) and for 1% lidocaine. "Responsiveness" is averaged for all animals (100=full pain response in all animals; 0%=no pain response in all animals). This figure shows the responsiveness as a function of time after treatment. In the period of 1 to 2.5 hours after injection the animals were rendered 8% responsive to the pain stimulus. The effect persists during the times that the lidocaine effect had worn off (cf. Figure 2).

Half-responsiveness was observed 70 hours after injection. The effect slowly wears off, with 100% responsiveness observed after approximately 140 hours, i.e., about six days.

Figure 4 shows the dependence of the initial responsiveness as a function of the dose. Figure 5 shows that the half-time for return to 50% responsiveness and shows a sigmoidal dependence on the dose of methoxyfluorane microdroplets, reaching a maximal half-time of 70 hours. Both the initial responsiveness effects and the halftime effects depend on the microdrop concentration in a graded manner consistent with the proposed mechanism of action: Large doses create large reservoirs of anesthetic within the tissue which must be removed before responsiveness to pain stimuli can be observed. Smaller doses can be used to create marginal anesthesia for a shorter time. In the latter case the injected dose of microdroplets does not have sufficient reservoir capacity to saturate the tissue. The maximal half-time for return of responsiveness of approximately 70 hours observed at maximal dose is believed to reflect the time that it takes the vesicles to be cleared from the tissue via the lymphatics.

### Example 2

Example 1 was repeated this time using 6.7% n-heptane as the anesthetic and similar results were obtained.

### Example 3

Example 1 was repeated this time using microdroplets with a 1:1 mixture of n-dibutyl ether and chloroform as the organic phase (6.7%) but the anesthesia was of short duration (1―2 hours). This correlates with the increased volatility and water solubility of these agents which give more rapid removal via the blood. The n-dibutyl ether chloroform microdroplets were shown to be effective in solubilizing benzocaine, but no increased efficacy of anesthesia was observed.

### Example 4

Lecithin coated methoxyfluorane microdropets were injected into the hind leg muscle of the rat (2.0 ml total dose) and this resulted in immobilization of its hind quarters for one day. Controlled injections of lidocaine gave only short-duration immobilization (approximately two hours).

### Example 5

Microdroplets were prepared as described in Example 1 except that the organic phase consisted of 6.7% mineral oil and the phospholipid monolayer consisted of didocecanoyl (dilauryl) lecithin (12.8 mg/ml). The microdroplets were found to be stable at 37°C *in vitro* for over a month. The microdroplets were injected into the tails of two rats and no toxic effects were observed. Local anesthesia was not observed, in accordance with expectations since mineral oil lacks anesthetic potency.

### Example 6

Microdroplets were prepared as described in Example 1 except that the organic phase consisted of 2.42% methoxyfluorane, 2.42% n-dibutyl ether and 1.8% mineral oil solubilizing 1.8 mg/ml benzocaine and the phospholipid monolayer consisted of didocecanoyl (dilauryl) lecithin (12.8 mg/ml). The microdroplets were found to be stable at 37°C *in vitro* for over a month. The microdroplets were injected into the tails of two rats and no toxic effects were observed. Local anesthesia was observed with kinetics similar to that given in Figures 4 and 5 for 2.4% methoxyfluorane.

## Claims

1. An aqueous suspension of microdroplets of from 20 nm to 10µm in diameter consisting essentially of a sphere of:
(i) a substantially Water-insoluble general or inhalation anaesthetic in liquid form; and
(ii) a phospholipid monolayer surrounding the anaesthetic;
the suspension being free of a fat or oil of vegetable or animal origin; for use in a method of inducing anaesthesia at the site at which anaesthesia is desired in a human or animal body.

2. An injectable pharmaceutical composition comprising a microdroplet according to claim 1 together with a pharmaceutically acceptable injectable vehicle.

3. Use of microdroplets according to claim 1 wherein the anaesthetic is a normally volatile known general or inhalation anaesthetic in liquid form for the manufacture of a medicament for use in producing local anaesthesia in a warm-blooded animal by injection.

4. The use according to claim 3 wherein the anaesthetic is a fluoro-halo-hydrocarbon compound.

5. The use according to claim 4 wherein the fluoro-halo-hydrocarbon compound is 1-bromo-1-chloro-2,2,2-trifluoroethane, 1-chloro-2,2,2-trifluoroethyl difluoromethyl ether, 2-chloro-1,1,2-trifluoroethyl difluoromethyl ether or 2,2-dichloro-1,2-difluoroethyl methyl ether.

6. The use according to claim 5 wherein the fluoro-halo-hydrocarbon compound is 2,2-dichloro-1,1-difluoroethyl methyl ether.

7. The use according to claim 3 wherein the medicament is designed for intradermal injection.

8. The use according to claim 3, wherein the medicament is designed for intramuscular injection.

9. Use of a microdroplet according to claim 1 for the manufacture of a medicament for use in inducing anaesthesia at the site at which anaesthesia is desired.

## Patentansprüche

1. Wäßrige Suspension von Mikrotropfen mit einem Durchmesser von 20 nm bis 10 µm, die im wesentlichen aus einer Sphäre aus
(i) einem im wesentlichen in Wasser unlöslichen Allgemein- oder Inhalationsanästhetikum in flüssiger Form und
(ii) einer Phospholipidmonoschicht um das Anästhetikum herum besteht, wobei die Suspension frei von Fett oder Öl pflanzlichen oder tierischen Ursprungs ist, zur Verwendung in einem Verfahren zur Induzierung von Anästhesie an dem Ort, an dem die Anästhesie in einem menschlichen oder tierischen Körper gewünscht ist.

2. Injizierbare pharmazeutische Zusammensetzung, die Mikrotropfen gemäß Anspruch 1 zusammen mit einem pharmazeutisch akzeptablen injizierbaren Träger umfaßt.

3. Verwendung von Mikrotropfen gemäß Anspruch 1, bei der das Anästhetikum ein normalerweise flüchtiges bekanntes Allgemein- oder Inhalationsanästhetikum in flüssiger Form ist, für die Herstellung eines Medikaments zur Erzeugung einer Lokalanästhesie in einem Warmblüter durch Injektion.

4. Verwendung nach Anspruch 3, bei der das Anästhetikum eine Fluor-Halogen-Kohlenwasserstoffverbindung ist.

5. Verwendung nach Anspruch 4, bei der die Fluor-Halogen-Kohlenwasserstoffverbindung1-Brom-1-chlor-2,2,2-trifluorethan, 1-Chlor-2,2,2-trifluorethyldifluormethylether, 2-Chlor1,1,2-trifluorethyldifluormethylether oder 2,2-Dichlor-1,2-difluorethermethylether ist.

6. Verwendung nach Anspruch 5, bei der die Fluor-Halogen-Kohlenwasserstoffverbindung2,2-Dichlor-1,1-difluorethylmethylether ist.

7. Verwendung nach Anspruch 3, bei der das Medikament zur intradermalen Injektion bestimmt ist.

8. Verwendung nach Anspruch 3, bei der das Medikament zur intramuskulären Injektion bestimmt ist.

9. Verwendung von Mikrotropfen gemäß Anspruch 1 für die Herstellung eines Medikaments zur Verwendung bei der Induzierung von Anästhesie an dem Ort, an dem die Anästhesie gewünscht wird.

## Revendications

1. Suspension aqueuse de microgouttelettes de 20nm à 10µm de diamètre consistant essentiellement en sphères :
(i) d'un anesthésique général ou d'inhalation, essentiellement insoluble dans l'eau, sous forme liquide, et
(ii) d'une mono-couche de phospholipide entourant l'anesthésique,
la suspension étant exempte de produit gras ou d'huile d'origine végétale ou animale, destinée à être utilisée dans une méthode induisant une anesthésie sur le site où l'on souhaite anesthésier un être humain ou animal.

2. Composition pharmaceutique injectable comprenant une microgouttelette selon la revendication 1 dans un véhicule injectable pharmaceutiquement acceptable.

3. Utilisation de microgouttelettes selon la revendication 1 dans laquelle l'anesthésique est un anesthésique général ou d'inhalation connu, généralement volatil, sous forme liquide, pour la préparation d'un médicament destiné à provoquer une anesthésie locale par injection chez un animal à sang chaud.

4. Utilisation selon la revendication 3 dans laquelle l'anesthésique est un composé fluoro-halo-hydrocarboné.

5. Utilisation selon la revendication 4 dans laquelle le composé fluoro-halo-hydrocarboné est le 1-bromo-1-chloro2, 2, 2-trifluoroéthane, le 1-chloro-2, 2, 2-trifluoroéthyl difluorométhyl éther, le 2-chloro-1, 1, 2-trifluoro éthyl difluorométhyl éther ou le 2,2-dichloro-1,2-difluoroéthyl méthyl éther.

6. Utilisation selon la revendication 5 dans laquelle le composé fluoro-halo-hydrocarboné est le 2, 2-dichloro-1, 1-difluoroéthyl méthyl éther.

7. Utilisation selon la revendication 3 dans laquelle le médicament est destiné à une injection intradermale.

8. Utilisation selon la revendication 3 dans laquelle le médicament est destiné à une injection intramusculaire.

9. Utilisation d'une microgouttelette selon la revendication 1 pour la préparation d'un médicament destiné à induire une anesthésie sur le site où l'on souhaite anesthésier.
